# EUROPEAN PATENT APPLICATION

(11) **EP 2 505 589 A1**
(43) Date of publication of application: **03.10.2012**
(21) Application number: 11160903.8
(22) Date of filing: 01.04.2011
(51) Int. Cl.: C07D 498/04, A61K 31/4188, A61P 35/00

(54) **Novel sphingolipid heterocyclic compounds as modulators of sphingolipid signaling and uses thereof**

(71) Applicant: Johann Wolfgang Goethe-Universität Frankfurt am Main, 60325 Frankfurt am Main (DE); Universität Bern, 3012 Bern (CH)
(72) Inventor: Stark, Holger, 61348, Bad Homburg (DE); Zivkovic, Aleksandra, 60438, Frankfurt am Main (DE); Huwiler, Andrea, 3011, Bern (CH); Pfeilschifter, Josef, 60594, Frankfurt am Main (DE)
(74) Representative: Krauss, Jan

(57) **Abstract**

The present invention relates to new sphingolipid heterocyclic compounds as modulators of sphingolipid signaling and uses thereof as pharmaceutically active agents, suitable for treating proliferative, degenerative, infectious, and other diseases. 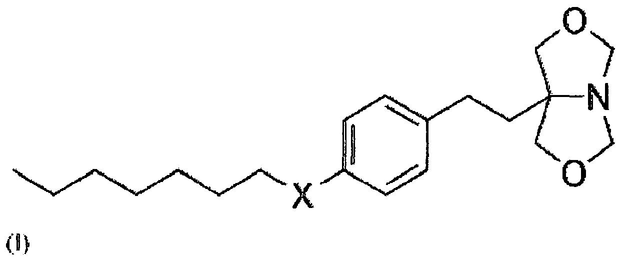 wherein X is selected from a methylene group (CH₂) and oxygen (O);
and pharmaceutically acceptable salts thereof.

## Description

The present invention relates to new sphingolipid oxalzolo-oxazole-compounds as modulators of sphingolipid signaling and uses thereof as pharmaceutically active agents, suitable for treating proliferative, degenerative, infectious, and other diseases. For the purposes of the present invention, all references as cited herein are incorporated by reference in their entireties.

### Background of the Invention

During the past decade there has been an enormous increase in research on sphingolipids due to discoveries that implicated members of this group in signal transduction processes [reviewed in Fernandis et al, Curr. Opin. Lipidol. 18: 121-8, (2007); Levade et al, Biochim. Biophys. Acta 1438, 1-17 (1999); Mathias et al, Biochem. J. 335, 465-480 (1998); Perry et al, Biochim. Biophys. Acta 1436, 233-243 (1998); Riboni et al, Prog. Lipid Res. 36, 153-195 (1997)].

The role of sphingolipids in signal transduction [reviewed in Eyster KM Adv Physiol Educ. 31: 5-16, (2007); L. Zheng et al Biochim Biophys Acta. 1758: 1864-84, (2006); Riboni et al, Prog. Lipid Res. 36, 153-195 (1997); and A. Huwiler et al Biochim Biophys Acta. 1485, 63-99 (2000)] has been extensively studied, and was proposed to operate through the "sphingomyelin cycle". According to this hypothesis, a variety of stress-inducing factors including proinflammatory cytokines, cell-damaging agents, chemotherapeutics, differentiation-inducing substances, and reactive nitrogen and oxygen species are able to activate a plasma membrane-bound sphingomyelinase that hydrolyzes sphingomyelin giving rise to ceramide. This sphingolipid, in turn, can either activate or inhibit intracellular targets finally triggering cellular responses, such as cell killing by apoptosis, inhibition of cell proliferation, cell differentiation and inflammatory reactions [A. Huwiler et al Biochim Biophys Acta. 1485, 63-99 (2000)]. Of particular interest are also reports that short-chain cell-permeable (e.g., C2 or C6) ceramides evoke biological responses that lead to cell killing. Other studies, using the degradation product of ceramide - sphingosine - have shown its critical effects on cell growth and viability. In general, multiple therapeutic indications have been proposed for the members of the sphingolipid signaling module. Compounds with some relationships to sphingosine are of special interest as they form one of the last step mediators generated in this cascade.

The phosphorylated form of sphingosine, i.e., sphingosine-1-phosphate has been shown to act either extracellularly via S1P-receptors, or intracellularly via still poorly defined targets in order to activate a variety of signaling cascades including the classical mitogen-activated protein kinase (MAPK) cascade, the protein kinase B/Akt cascade, and the TGFβ/Smad cascade. In addition, various phospholipases including PLA₂, PLC, and PLD are activated. Thus, sphingosine-1-phosphate acts as a direct as well as an indirect messenger in lipid signalling. Di- or tri- methylated sphingosines were shown to inhibit growth of cancer cells [Endo et al., Cancer Research 51, 1613-8, (1981)].

US 2008/0305063 describes compounds of the general formula 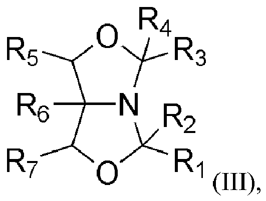 wherein R₁, R₂, R₃, R₄ independently can be moieties of the general formula R₁-C(=O)-R₂ and/or R₃-(C=O)-R₄, wherein R₁ and R₂ and/or R₃ and R₄ at the same time can not all be hydrogen. R₆ can be H or alkyl, which can be substituted, e.g. with OH-groups and/or amino groups and/or wherein up to eight of non-adjacent CH₂ groups can be replaced by O. R₅ and R₇ can be H. The compounds are used as fragrances.

US 7,598,282 describes neuroprotective compounds of the general formula 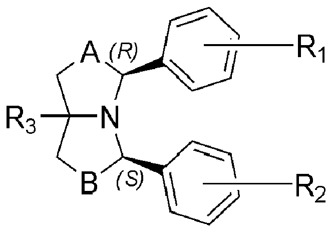 wherein A and B are sulfur or oxygen; wherein R₁ and R₂ independently are halogen, alkyl, alkoxy, haloalkyl; and wherein R₃ is alcohol.

US 2006-0281709 describes anti fibrotic agents containing a sphingosine-1-phosphate receptor agonist or sphingosine 1-phosphate as active ingredient, and use thereof in a method for treating a fibrosis.

Rosen et al (in: Rosen H, Gonzalez-Cabrera P, Marsolais D, Cahalan S, Don AS, Sanna MG. Modulating tone: the overture of SIP receptor immunotherapeutics. Immunol. Rev. 2008; 223:221-35) describe the five distinct high affinity G-protein-coupled receptor subtypes with the shared ligand sphingosine-1-phosphate (SIP), which are capable of modulating immune responses in vivo, with the impact on the immune response being very different from classical immunosuppressants. Pharmacological subversion of the SIP rheostat is described as proving to be clinically efficacious in multiple sclerosis.

The immunomodulatory agent FTY720 (fingolimod) is used as a therapeutic for the treatment of multiple sclerosis, and is evaluated for use in immunomodulatory diseases. Fingolimod primarily stimulates sphingosine-1-phosphate receptors as an agonistic prodrug, but secondary to that, FTY720 desensitizes specifically the SIP receptor subtype, thus acting as a functional antagonist. This latter effect was also proposed to be responsible for its therapeutic effect as immunosuppressant. The structure of FTY720 is as follows: 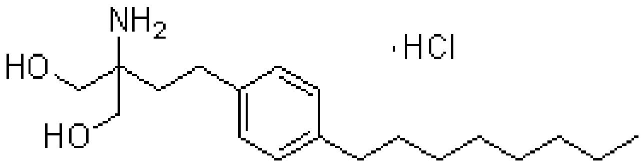 2-Amino-2[2-(4-octylphenyl)ethyl]propane-1,3-diol compounds are disclosed in EP-A-0627406.

WO 2010/055027 relates to salts, e.g. crystalline salts, of the compound FTY720, and to the use thereof. The compounds have been found to be useful as immunosuppressants. Accordingly, the compounds may be useful in the treatment or prevention of various autoimmune conditions, including multiple sclerosis. A particular compound in this class is FTY720, which may be obtained in the form of the free base or a hydrochloride salt.

Nagaoka et al. (in: Nagaoka Y, Otsuki K, Fujita T, Uesato S. Effects of phosphorylation of immunomodulatory agent FTY720 (fingolimod) on antiproliferative activity against breast and colon cancer cells. Biol. Pharm. Bull. 2008 ;31(6):1177-81) describe FTY720 (fingolimod), an immunosuppressant, which becomes biologically activated by phosphorylation into FTY720-1-phosphate (FTY720-P), which is a high-affinity agonist for four out of the five SIP receptor subtypes. It is mentioned that FTY720 has an antitumor activity; nevertheless, the association between the phosphorylation of FTY720 and the growth inhibition of FTY720 against cancer cells is speculative and not understood. In the study by Nagaoka et al., the effects of FTY720, sphingosine, and their related compounds on the proliferation of human breast cancer cell lines (MCF-7, MDA-MB-231 and Sk-Br-3) and human colon cancer cell lines (HCT-116 and SW620) were investigated. Non-phosphorylated FTY720, sphingosine and an FTY720 derivative, ISP-I-55, showed significant growth inhibition in these cells. In contrast, the phosphorylated derivatives, FTY720-P and sphingosine-1-P, as well as a phosphinane FTY720 derivative, cFTY720-P, did not inhibit the growth of the cells in the concentration tested, whereas FTY720-P and sphingosine-1-P slightly induced the growth of MCF-7 cells. Combining FTY720 with dimethylsphingosine, a sphingosine kinase inhibitor, augmented the inhibitory effect of FTY720. These results indicated that the antiproliferative activity of FTY720 does not result from its phosphorylation, either endogenously or exogenously.

It is an object of the present invention to provide novel compounds which are in particular suitable for treating disorders associated with increased cell proliferation and/or increased angiogenesis such as cancer and retinopathies. It is another object of the present invention to provide novel compounds and combination of compounds which are suitable in treating inflammatory diseases, including glomerulonephritis, autoimmune-mediated diseases, in particular graft versus host disease (GVHD), as well as other pathological states associated with increased prostaglandin formation such as pain.

It is a further object of the present invention to provide novel sphingolipid analogs which do not have prodrug-characteristics and thus have a less complex pharmacological profile than fingolimod (FTY720).

It is a further object of the present invention to provide novel sphingolipid analogs with a potential for treating neurodegenerative disorders, metabolism-associated conditions, and infectious diseases.

The invention, in a first aspect thereof, provides a compound of formula (I): 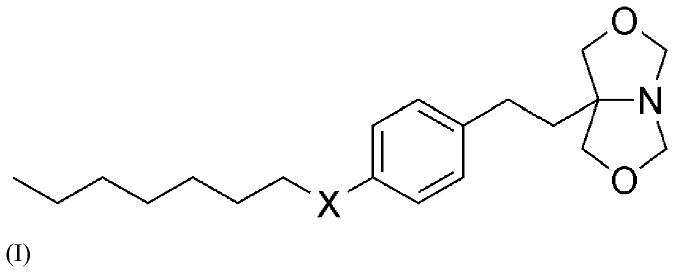 wherein X is selected from a methylene group (CH₂) and oxygen (O); and pharmaceutically acceptable salts thereof.

New sphingolipid analogs have now been synthesized, exhibiting surprisingly strong therapeutical effects. The present invention thus relates to sphingolipid-modulators that do not have prodrug characteristics, and therefore have a less complex pharmacological profile than other sphingolipid-modulators, such as, for example, fingolimod (FTY720). The compounds according to the present invention comprise lipophilic structures with a high similarity to FTY720. Therefore, the compounds according to the present invention represent a structurally related class of compounds, but lack prodrug characteristics, while still being able to efficiently influence the sphingolipid signaling. The lack of prodrug characteristics has several advantages with respect to the pharmacological handling, such as, for example, the dosing and effective administration of said drug, since the physiological, and therefore variable, metabolic step of converting the prodrug is bypassed and thus avoided. Therefore, the compounds according to the present invention should exhibit to be more predictable in their pharmacological effect(s).

Preferred is a compound according to the present invention, wherein X is CH₂ according to the following formula (II):

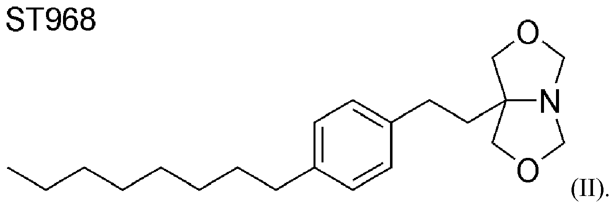

Also preferred is a compound according to the present invention, wherein X is O according to the following formula (III):

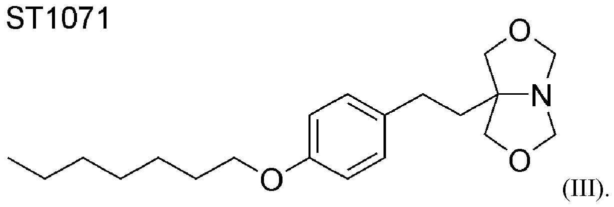

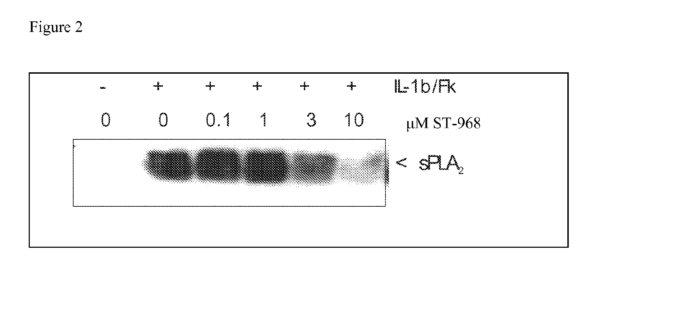

In biological assays as performed by the inventors, the compounds of the present invention, in particular ST 968 and ST1071 exhibited agonistic potential for one or several of the SIP receptor subtypes. Furthermore, anti-inflammatory effects were found. In addition, the compounds according to the invention, and in particular compounds ST 968, and ST 1071 (cf. Fig. 2), inhibited and/or reduced the colony formation in colon cancer cells (HCT-116). The compounds according to the present invention are thus expected to be potent growth inhibiting agents on a variety of cancer cell lines, including drug-sensitive and drug-resistant cells, alone or in combination with other anti-cancer drugs.

Another aspect of the present invention relates to a method for treating diseases, wherein said diseases are characterized by an imbalance in sphingolipid signaling comprising administering to a subject in need thereof a therapeutically effective amount of a compound of formula (I) according to the present invention.

In the subject invention a "therapeutically effective amount" is any amount of a compound or composition which, when administered to a subject suffering from a disease against which the compounds are effective, causes reduction, remission, or regression of the disease.

A "subject" is a vertebrate, mammal, domestic animal or human being.

In the practice of this invention the "pharmaceutically acceptable vehicle" is any physiological vehicle known to those of ordinary skill in the art useful in formulating pharmaceutical compositions.

The invention further relates to a pharmaceutical composition comprising as active ingredient at least one compound according to the present invention, and optionally further comprising pharmaceutically acceptable carrier, adjuvant or diluent. The compounds of the invention are generally provided in the form of pharmaceutical compositions. Said compositions are for use by injection, dermal as well as topical application, or by oral uptake. Patches may be used for a prolonged drug release, as well as other suitable pharmaceutical formulations.

The pharmaceutical compositions of the invention generally comprise a buffering agent, an agent which adjusts the osmolarity thereof, and optionally one or more carriers, excipients and/or additives as known in the art, e.g., for the purposes of adding flavors, colors, lubrication, or the like to the pharmaceutical composition.

Each carrier should be both pharmaceutically and physiologically acceptable in the sense of being compatible with the other ingredients and not injurious to the subject to be treated. While formulations include those suitable for rectal, nasal, preferred formulations are intended for oral or parenteral administration, including intramuscular, intradermal, subcutaneous and specifically intravenous administration. The formulations may conveniently be presented in unit dosage form and may be prepared by any methods known in the art of pharmacy.

The present invention also encompasses pharmaceutically acceptable salts of the present compounds. Such salts include pharmaceutically acceptable addition of salts, pharmaceutically acceptable metal salts, ammonium, and alkylated ammonium salts. Acid addition salts include salts of inorganic acids as well as organic acids. Representative examples of suitable inorganic salts include hydrochloric, hydrobromic, hydroiodic, phosphoric, sulfuric, nitric acids and the like. Representative examples of suitable organic acids include, formic, acetic, trichloroacetic, trifluoroacetic, propionic, benzoic, cinnamic, citric, fumaric, glycolic, lactic, maleic, malic, methansulfonic, ethansulfonic, aspartic, stearic, palmitic, EDTA, glycolic, glutamic, malonic, mandelic, oxalic, picric, salicylic, siccinic, sulfonic, gluconic, citraconic, tartaric, ascorbic, bismethylene salicylic, ethynditamic, benzene sulfonic, p-toluene sulfonic acids and the like. Further examples of pharmaceutically acceptable inorganic and organic acid addition salts include the pharmaceutically acceptable salts listed in e.g., S. M. Berge, L. D. Bighley, D. C. Monkhouse, J. Pharm. Sci. 1977, 66, 1-19, and P. H. Stahl, C: G: Wermuth, Handbook of pharmaceutical salts: Properties, selection and use. 2002, Verlag Helvetica Chimica Acta, Zürich; ISBN: 3-906390-26-8. Examples of metal salts include lithium, sodium, potassion, magnesium salts and the like. Examples of ammonium, alkylted ammonium salts include ammonium, methylammonium, dimethylammonium, trimethylammonium, ethylammonium, hydrixyethylammnonium, diethylammonium, butylammonium, tetramethylammnonium salt and the like.

Also intended as pharmaceutically acceptable acid addition are the hydrates, which the present compounds are able to form.

The acid addition may be obtained as direct products of compound synthesis. In the alternative, the free base may be dissolved in a suitable solvent containing the appropriate acid, and the salt isolated by evaporating the solvent or otherwise separating the salt and solvent.

The compounds of the present invention may form solvates with standard low molecular weights solvents using methods well known to the person skilled in the art. Such solvates are contemplated as being within the scope of the present invention.

Carriers may include starch and derivatives thereof, cellulose and derivatives thereof, e.g., microcrystalline cellulose, xanthane gum, and the like. Lubricants may include hydrogenated castor oil and the like.

As used herein "pharmaceutically acceptable carrier" includes any and all solvents, dispersion media, and coatings, not harmful to the subject.

The pharmaceutical compositions formed by combining the novel compounds of the present invention and the pharmaceutical acceptable carriers are then readily administered in a variety of dosage forms suitable for the disclosed routes of administration. The formulations may conveniently be presented in unit dosage form by methods known in the art of pharmacy. Formulations of pharmaceutical compositions according to the present invention suitable for oral administration may be presented as discrete units such as capsules or tablets, each containing a predetermined amount of the active ingredient, and which may include a suitable excipient. These formulations may be in the form of powder or granules or melted solution, as a solution or suspension in an aqueous solution or non-aqueous liquid, or as an oil-in-water or water-in-oil emulsion.

If desired, the pharmaceutical composition of the invention may comprise a compound according to the invention in combination with one or more pharmacologically active substances. Antibacterial and antifungal agents may be included. The use of such media and agents for pharmaceutical active substances is well known in the art.

The invention also encompasses active metabolites of the present compounds. Such metabolites include those formed by chemical reactions a well as by enzymatic reactions in the body. The definition of active metabolites includes the formation of phase I as well as phase II metabolites as different conjugated derivatives.

The compounds of the invention may be any pharmaceutical acceptable salt thereof including different or special polymorphic forms.

The pharmaceutical compositions may be specifically formulated for administration by any suitable route such as the oral, rectal, nasal, pulmonal, topical (incl. buccal and sublingual), transdermal, itracisternal, intraperitoneal, intravitreal, vaginal and parenteral (incl. subcutaneous, intramuscular, intrathecal, intravenous and intradermal) route. The oral and the transdermal routes are preferred and the oral route is most preferred. It will be appreciated that the preferred route will depend on the general condition and age of the subject to be treated, the nature of the condition to be treated and the active ingredient chosen.

In a preferred embodiment of the invention, the compounds are formulated for oral administration.

Pharmaceutical compositions for oral administration may be in a solid dosage form such as capsules, tablets, dragees, pills, lozenges, powders and granules. Solid dosage forms can include one or more substances which may also act as flavoring agents, lubricants, solubilizers, suspending agents, fillers, glidants, compression aids, binders or tablet-disintegrating agents. In powders, the vehicle is a finely divided solid which is in admixture with the finely divided compound according to the invention. In tablets, the compound according to the invention is mixed with a vehicle having the necessary compression properties in suitable proportions and compacted in the shape and size desired. The powders and tablets preferably contain up to 99% of the compound of the invention. Suitable solid vehicles include, for example, calcium phosphate, magnesium stearate, talc, sugars, lactose, dextrin, starch, gelatin, cellulose, polyvinylpyrrolidine, low melting waxes and ion exchange resins.. The amount of solid carrier will vary widely but will usually be from about 25 to about 1000 mg.

Where appropriate, solid dosage forms can be prepared with coatings such as enteric coatings or they can be formulated so as to provide controlled release of the active ingredient such as sustained or prolonged release or bolus application according to the methods well known in the art.

A typical preferred tablet, which may be prepared by conventional tableting techniques (e.g. by compression), may contain:

| Core: | | |
|---|---|---|
| Active compounds (as free compound or salt thereof) | | 5.0 mg to 10.0 mg |
| Lactosum Ph. Eur. | | 67.8 mg |
| Cellulose, microcryst. (Avicel) | | 31.4 mg |
| Amberlite® IRP88* | | 1.0 mg |
| Magnesii stearas Ph. Eur. | | q.s. |
| Coating: | | |
| Hydroxypropyl methylcellulose | approx. | 9 mg |
| Mywacett 9-40 T** | approx. | 0.9 mg |

| | | |
|---|---|---|
| * Polacrillin potassium NF, Tablet disintegrant, Rohm and Haas ** Acylated monoglyceride used as plasticizer for film coating | | |

Pharmaceutical compositions for oral administration may alternatively be in a liquid form. A liquid carrier may be in the form of solutions, suspensions, emulsions, syrups, elixirs and pressurized compositions. The compound of the invention can be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid vehicles for oral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil).

In another embodiment of the invention the compounds are formulated for parenteral administration.

Preferably liquid compositions are used parenterally. When this is the case the liquid forms, and particularly the liquid vehicles discussed above may be used. Furthermore, for parenteral administration, the vehicle can also be an oily ester such as ethyl oleate and isopropyl myristate. Sterile liquid vehicles are useful in sterile liquid form compositions for parenteral administration.

Liquid pharmaceutical compositions that are sterile solutions or suspensions can be utilized for other preferred routes of administration. For example, liquids may be used for intramuscular, intrathecal, epidural, intraperitoneal, intravitreal, or subcutaneous injection. Sterile solutions can also be administered intravenously. The compounds may be prepared as a sterile solid composition which may be dissolved or suspended at the time of administration using sterile water, saline, or other appropriate sterile injectable medium. Vehicles include necessary and inert binders, suspending agents, lubricants, flavorants, sweeteners, preservatives, dyes, and coatings.

Other suitable administration forms include suppositories, sprays, ointments, crèmes, gels, inhalates, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administration in one or more dosages such as one to three dosages. The exact dosage will depend upon the frequency and mode of application, the sex, age, weight and general condition of the subject treated, the nature and severity of the condition treated and any concomitant disease to be treated and other factors evident to those skilled in the art.

The pharmaceutical composition according to the invention may conveniently be presented in unit dosage form by methods known to those skilled in the art. A typical unit dosage form for oral administration one or more times per day, such as 1 to 3 times per day, contain 0.05 to about 1000 mg, preferably from about 0.1 to about 500 mg, more preferred from about 0.5 to about 3000 mg, more preferably about 10 to about 1000 mg, and most preferably about 10 to about 500 mg.

For parenteral routes, such as intravenous, intrathecal, intramuscular, intra-peritoneal and similar administration, typically doses are in the order of about half the dose employed for oral administration.

The compounds of the invention are generally utilized as the free substance or as a pharmaceutically acceptable salt thereof. One example is an acid addition salt of a compound having the utility of a free base. When a compound of the present invention contains a free base such salts are prepared in a conventional manner by treating a solution or suspension of a free base of the present invention with a chemical equivalent of a pharmaceutical acceptable acid, for example inorganic or organic acids. Representative examples are mentioned above. Physiologically acceptable salts of a compound with a hydroxyl group include the anion of said compound in combination with a suitable cation such as sodium or ammonium ion.

Another preferred pharmaceutical formulation is preferably used for administration by injection, including intravenous injection.

For parenteral administration, solutions of the novel compounds of the present invention in sterile aqueous or non-aqueous solution, aqueous propylene glycol or sesame or peanut oil may be employed. Such aqueous solutions should be buffered if necessary and the liquid diluted first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

The pharmaceutical forms suitable for injection use include sterile aqueous solutions or dispersions and sterile powders for the extemporaneous preparation of sterile injectable solutions or dispersions. In all cases the form must be sterile and must be fluid to the extent that easy syringeability exists. It must be stable under the conditions of manufacture and storage and must be preserved against the contaminating action of microorganisms, such as bacteria and fungi. The carrier can be solvent or dispersion medium containing, for example, water, ethanol, polyol (for example, glycerol, propylene glycol, and liquid polyethylene glycol, and the like), suitable mixtures thereof, and vegetable oils. The proper consistency can be maintained, for example, by the use of a coating, such as lecithin, by the maintenance of the required particle size in the case of dispersion, and by the use of surfactants.

Sterile injectable solutions are prepared by incorporating the active compounds in the required amount in the appropriate solvent with several of the other ingredients enumerated above, as required, followed by filter sterilization. Generally, dispersions are prepared by incorporating the various sterilized active ingredients into a sterile vehicle which contains the basic dispersion medium and the required other ingredients from those enumerated above.

In the case of sterile powders for the preparation of the sterile injectable solutions, the preferred method of preparation are vacuum-drying and freeze drying techniques which yield a powder of the active ingredient plus any additional desired ingredient from a previously sterile-filtered solution thereof.

The preparation of pharmaceutical compositions is well known in the art and has been described in many articles and textbooks, see e.g., Remington's Pharmaceutical Sciences, Gennaro A.R. ed., Mack Publishing Company, Easton, Pennsylvania, 1990, and especially pages 1521-1712 therein.

Additives may also be designed to enhance uptake of the active agent across cell membranes. Such agents are generally agents that will enhance cellular uptake of the molecules of the invention. For example, the compounds of the invention may be enclosed within liposomes. The preparation and use of liposomes, e.g., using particular transfection reagents, is well known in the art. Other methods of obtaining liposomes include the use of Sendai virus or of other viruses.

The dose of the active agent may vary to the individiual therapeutically effective amount. The dose would generally depend on the disease, the state of the disease, age, weight and sex of the patient, and is to be determined by the attending physician.

A number of methods of the art of molecular biology are not detailed herein, as they are well known to the person of skill in the art. Textbooks describing such methods are e.g., Sambrook et al., Molecular Cloning, A Laboratory Manual, Cold Spring Harbor Laboratory, ISBN: 0879693096, 1989; Current Protocols in Molecular Biology by F. M. Ausubel, ISBN: 047150338X, John Wiley & Sons, Inc. 1988; and Short Protocols in Molecular Biology by F. M. Ausubel et al. (eds.) 3rd ed., John Wiley & Sons, ISBN: 0471137812, 1995. Furthermore, a number of immunological techniques are not in each instance described herein in detail, as they are well known to the person of skill in the art (see, for example, Current Protocols in Immunology, Coligan et al. (eds), John Wiley & Sons. Inc., New York, NY).

Another aspect of the present invention then relates to a compound according to the present invention, or the pharmaceutical composition according to the present invention for use in medicine, and the treatment of diseases.

The pharmaceutical compositions comprising a compound according to the present invention are thus particularly intended for the treatment of diseases that are related to imbalances of sphingolipid signaling and/or can be treated (that is at least the symptoms can be ameliorated) based on a modulation of sphingolipid signaling using the compounds according to the present invention, such as, for example, a disorder selected from the group consisting of proliferative disorders, angiogenesis-related disorders, neurodegenerative disorders, metabolism-associated conditions, infectious diseases, immunity-associated conditions, and pain.

Preferred is the pharmaceutical composition according to the present invention, wherein said proliferative disorder is cancer, or wherein said angiogenesis-associated disorders are retinopathies such as age-related macula degeneration or diabetic retinopathy, or wherein said neurodegenerative disorder is Alzheimer's disease or multiple sclerosis, or wherein said metabolism-associated condition is selected from diabetes, cystic fibrosis, and lipid storage diseases, or wherein said infectious disease is selected from the group consisting of viral infections, bacterial infections, fungal infections, and protozoal infections, or wherein said immunity-associated condition are autoimmune and inflammatory diseases, GVHD or allergy.

Another aspect of the present invention then relates to the pharmaceutical composition according to the present invention for killing of drug-sensitive and drug-resistant cancer cells, in particular so-called "multidrug-resistant" cancer cells.

Yet another aspect of the present invention then relates to the use of a compound according to the present invention in the preparation of a medicament for treating a disorder selected from the group consisting of proliferative disorders, neurodegenerative disorders, metabolism-associated conditions, infectious diseases, and immunity-associated conditions.

In yet a further aspect the invention relates to a method for treating a cell proliferative, particularly cancerous disease, specifically for killing of wild type and drug-resistant cancer cells in a patient in need of such treatment, comprising administering to said patient a therapeutically effective amount of a compound of formula (I) or of pharmaceutical composition comprising the same. In a preferred embodiment, a pharmaceutical composition comprising at least one compound of above formula (I) is used for the treatment of neurodegenerative disorders, metabolism- associated conditions, infectious diseases, and immunity-associated conditions in a patient in need of such treatment, comprising administering to said patient a therapeutically effective amount of a compound of formula (I) according to the present invention, or a pharmaceutically acceptable salt thereof.

Yet another aspect of the present invention relates to a method for the treatment, prevention and/or amelioration of a disease/disorder caused by or related to imbalanced sphingolipid signaling, the method comprising administering an amount of a pharmaceutical composition in accordance with the present invention that is effective to treat, prevent and/or ameliorate such a disease/disorder.

While the invention has been described using some specific examples, many modifications and variations are possible. It is therefore understood that the invention is not intended to be limited in any way, other than by the scope of the appended claims. The above and other characteristics and advantages of the invention will be more readily apparent through the following examples, and with reference to the appended drawings, wherein:

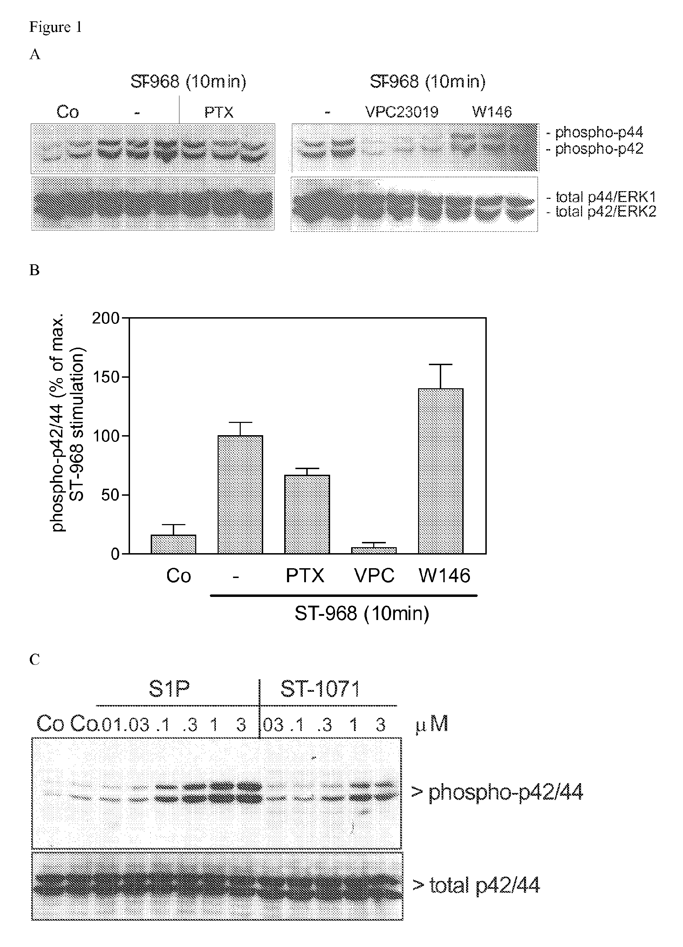

Fig. 1. shows the effect of pertussis toxin, and the S1P₁/S1P₃ receptor antagonists VPC23019 and W146 on ST-968-stimulated MAPK/ERK phosphorylation, and of ST-1071-stimulated MAPK/ERK phosphorylation in renal mesangial cells. (A+B): Confluent mesangial cells were pretreated for 16h with pertussis toxin (100 ng/ml), or for 30min with vehicle (-), VPC23019 (10µM) and W146 (10 µM) prior to stimulation for 10min with either vehicle (Co) or ST-968 (1µM). (C): Cells were stimulated for 10 min with either vehicle (Co) or the indicated concentrations (in µM) of SIP and ST-1071. Thereafter, cell lysates containing 30 µg of protein were separated by SDS-PAGE, transferred to nitrocellulose and subjected to Western blot analysis using antibodies against phospho-p42/44 (dilution of 1:1000; A, upper panel) and total p42/ERK2 and p44/ERK1 (dilution 1:6000 each; B, lower panel). Blots were stained by the ECL method according to the manufacturer's recommendation. Bands corresponding to the phospho-p42/44 and total ERKs were densitometrically scanned and evaluated (B). Results in B are depicted as % of ST-968 stimulation and are means ± S.D. (n=3).
Fig. 2. shows the effect of ST-968 on cytokine-triggered secretory phospholipase A₂ expression in renal mesangial cells. Cells were treated for 24h with either vehicle (-, 0) or with interleukin 1β (1 nM) plus forskolin (10 µM) (IL-1/Fk, +) in the absence (0) or presence of the indicated concentrations of ST-968. Thereafter, supernatants were collected and equal volumes were taken for protein precipitation using trichloroacetic acid. Equal aliquots were separated by SDS-PAGE, transferred to nitrocellulose and subjected to Western blot analysis using a monoclonal antibody against rat sPLA₂ at a dilution of 1:60. Blots were stained by the ECL method according to the manufacturer's recommendation. Data are representative of two independent experiments giving similar results.

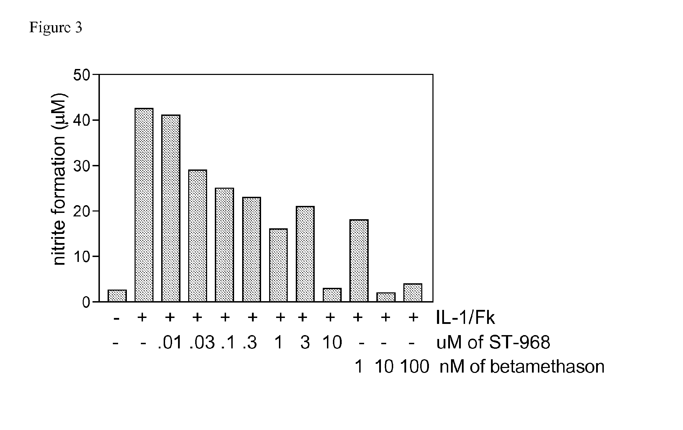

Fig. 3. shows the effect of ST-968 on cytokine-triggered nitric oxide release from renal mesangial cells. Cells were treated for 24h with either vehicle ( -) or with interleukin 1β (1 nM) plus forskolin (10 µM) (IL-1/Fk, +) in the absence or presence of the indicated concentrations of ST-968 (in µM), or betamethasone (in nM). Thereafter, supernatants were collected and nitrite was quantified by using the Griess reagent.

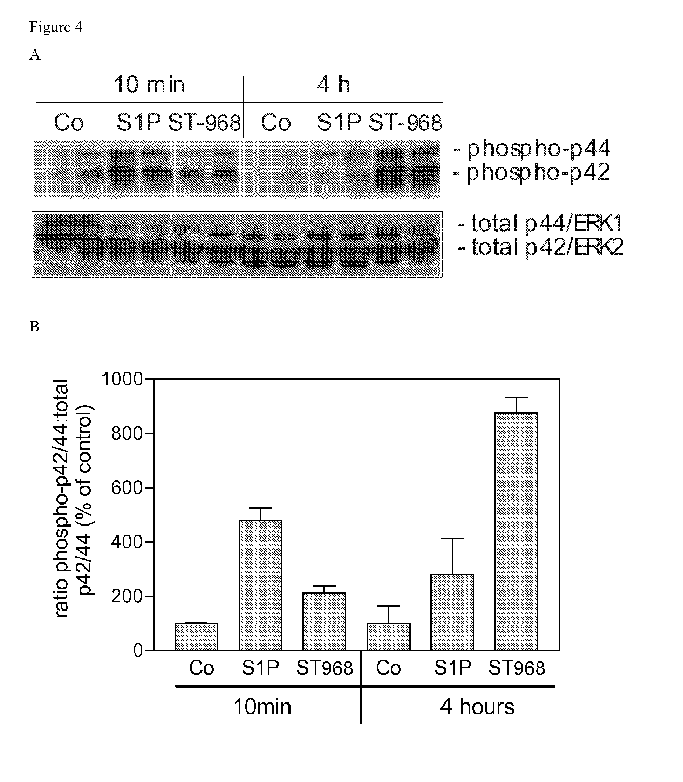

Fig. 4 shows the effect of short-term and long-term treatment with SIP and ST-968 on MAPK/ERK phosphorylation in the human endothelial cell line EA.hy 926. Confluent EA.hy 926 cells were treated either for 10min (left side) or for 4h (right side) with either vehicle (Co), SIP (1 µM), or ST-968 (1 µM). Thereafter, cell lysates containing 30 µg of protein were separated by SDS-PAGE, transferred to nitrocellulose and subjected to Western blot analysis using antibodies against phospho-ERK1/2 (dilution of 1:1000; A, upper panel), and total p42/ERK2 and p44/ERK1 (dilutions each 1:6000; B, lower panel). Blots were stained by the ECL method according to the manufacturer's recommendation. Bands corresponding to the phospho-ERKs were densitometrically evaluated and results are depicted as % of control and are means ± S.D. (n=2/3).

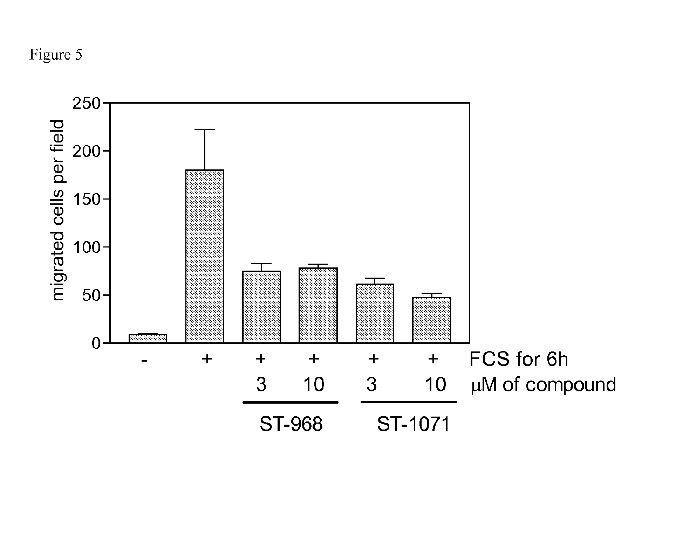

Fig. 5 shows the effect of ST-968 and ST-1071 on serum-stimulated migration of human endothelial cells. Serum-starved EA. hy 926 cells in 8 µM-polycarbonate inserts were treated for 6h with 10% serum in the absence or presence of the indicated concentrations of ST-968 or ST-1071. Migrated cells were stained with DAPI and counted in a fluorescence microscop. Results show the number of migrated cells per field and are means +/- S.D. (n=4).

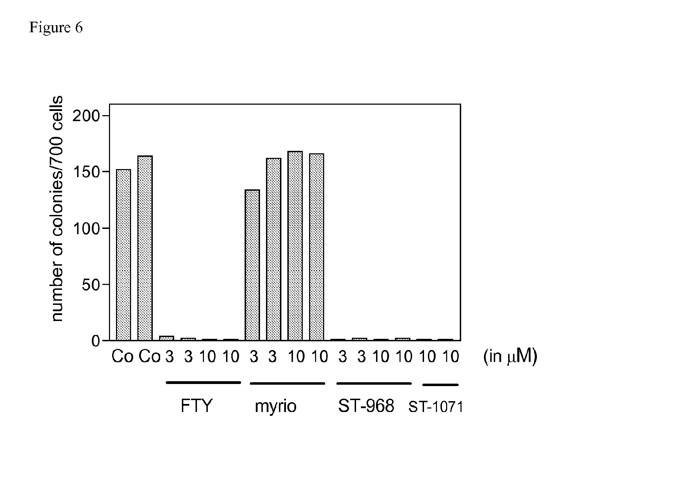

Fig. 6 shows the effect of the novel FTY720 derivatives and myriocin on colony formation of the human colon cancer cell line HCT-116. Cells were seeded at a density of 700 cells per 60mm-diameter dish in growth medium. After 24h, cells were treated with either vehicle (Co) or the indicated concentrations (in µM) of FTY720, myriocin (myrio), ST-968, and ST-1071, and further cultured for 10 days at 37°C. Thereafter, colonies were stained and quantified as described in the Methods Section. Data show the number of colonies per 700 seeded cells and are single values. The experiment shows duplicates and is representative of two independent experiments with similar results.

The following examples are only representative of techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

### EXAMPLES

The compounds provided herein are particularly useful in the treatment (as well as prevention and/or amelioration) of human diseases or disorders. Compounds provided herein have been scrutinized in specific biophysical/biochemical tests and have been further evaluated in cell-based disease/disorder models.

### Experimental procedures

The starting compound FTY720 and oxy analogues compound were prepared as previously reported in a literature (Fujita et al., J. Med. Chem. 2000. 43, 2946; Zivkovic and Stark, Tetrahedr. Lett. 2010, 51, 3769). Examples 1 and 3 describe the synthesis of the product under the reductive amination conditions and examples 2 and 3 the synthesis of the compounds ST968 and ST1071 using Dean-Stark-apparatus. (reductive amination: Darabantu et al., (2000a) Tetrahedron, 2000, 56(23), 3799-3816;; Darabantu et al. (2000b) Tetrahedron, 2000b, 56(23), 3785-3798; Darabantu (2004) Eur. J. Org. Chem., 2004, 12, 2644-2661

### Example 1

### Preparation of 7a-(4-octylphenethyl)tetrahydro-1H-oxazolo[3,4-c]oxazole (ST-968)

A mixture of FTY720 (400mg, 1.30mmol), ZnCl₂ (720 mg, 5.20mmol) and paraformaldehyde (160mg, 5.20mmol) in 15ml CH₂Cl₂ was stirred at RT for 90 min and then NaBH₄ (200mg, 5.20mmol) was added and the resulting mixture was stirred for additional hour. The reaction was quenched by addition of 25% NH₃ (10ml) stirred for additional 10min and organic layer was separated, dried over MgSO₄ and evaporated to dryness. Crude product was submitted to column chromatography (eluent: hexane/EtOAc 3/1). The product was isolated in 50% yield.

**¹H NMR:** δ [ppm] (400 MHz, MeOD-*d₄*) 7.15 (q, *J*=8.4Hz, 4H, H_{Ar}), 4.49 (d, *J*=6 Hz, 2H, -N-**CH₂**O-), 4.35 (d, *J*=6 Hz, 2H, -N-**CH₂**O-), 3.80 (d, *J*=8.8Hz, 2H, CH₂-O-**CH₂**-C), 3.64 (d, *J*=8.8Hz, 2H, CH₂-O-**CH₂**-C), 2.60(m, 4H, 2*Ar-**CH₂**, ), 1.85 (m, 2H, Ar-**CH₂**-CH₂-C(CH₂O-N-), 1.58 (m, 2H, Ar-**CH₂**-CH₂-(CH₂)₅-CH₃), 1.30 (m, 10H, Ar-CH₂-**(CH₂)₅**), 0.92 (t, *J*=6.9Hz, 3H, Ar-(CH₂)₇-**CH₃**);

| | |
|---|---|
| **ESI (+):** | 332.1 ([M+H]⁺); |
| **TLC:** | R_{f}= 0. 5 (hexan/EtOAc:3/1) |
| El. Analysis: | Calculated: C (76.09%), H(10.03%), N(4.52%); |
| | Found: C( 76.31%), H (9.84%), N( 4.23%); |

### Example 2

### Preparation of 7a-(4-octylphenethyl)tetrahydro-1H-oxazolo[3,4-c]oxazole (ST-968)

The mixture of Fingolimod (50mg) and paraformaldehyde (50mg) was dissolved in toluene (40ml) and catalytic amount of p-TSA was added. The reaction mixture was stirred under reflux 20h using Dean-Stark apparatus. The solvent was than evaporated to dryness and the crude product was partitioned between EtOAc and water. Organic phase was dried over MgSO₄ and evaporated to dryness. 50mg of pure ST968 were isolated with the analytical data already listed for example 1. The compound was crystallized also from diethylether as HCl salt (added HCl 2M Et₂O)-ST-968*HCl. Analytical data correspond to that of Example 1.

**¹H NMR:** δ [ppm] (400 MHz, MeOD-*d₄*)

7.12 (q, *J*=8.4Hz, 4H, H_{Ar}), 4.45 (d, *J*=6 Hz, 2H, -N-**CH₂**O-), 4.42 (d, *J*=6 Hz, 2H, -N-**CH₂**O-), 3.80 (d, *J*=8.8Hz, 2H, CH₂-O-**CH₂**-C), 3.64 (d, *J*=8.8Hz, 2H, CH₂-O-**CH₂**-C), 2.60(m, 4H, 2*Ar-**CH₂,** ), 1.85 (m, 2H, Ar-CH₂-**CH₂**-C(CH₂O-N-), 1.58 (m, 2H, Ar-CH₂-**CH₂**(CH₂)₅-CH₃), 1.30 (m, 10H, Ar-CH₂-**(CH₂)**₅), 0.92 (t, *J*=6.9Hz, 3H, Ar-(CH₂)₇-**CH₃**);

| | |
|---|---|
| **ESI (+):** | 332.1 ([M+H]⁺); |
| El. Analysis: | Calculated: C (68.55%), H(9.31%), N(3.81%); |
| | Found: C(68.42%), H (9.42%), N(3.67%); |

### Example 3

### Preparation of 7a-(4-(heptyloxy)phenethyl)tetrahydro-1H-oxazolo[3,4-c]oxazole

The compound ST-1071 was synthesized in the same manner as the compound ST-968 (see Example **1**).

**¹H NMR:** δ [ppm] (250 MHz, DMSO-d₆)

7.06 (d, *J*=8.5Hz, 2H, H_{Ar}), 6.77 (d, *J*=8.75Hz, 2H, H_{Ar}), 4.38 (d, *J*=5.75 Hz, 2H, -N-**CH**₂O-), 4.24 (d, *J*=5.75 Hz, 2H, -N-**CH**₂O-), 3.85 (t, *J*= 6.5Hz, Ar-O-**CH₂**-), 3.69 (d, *J*=8.75 Hz, 2H, CH₂-O-**CH₂**-C), 3.53 (d, *J*=8.75 Hz, 2H, CH₂-O-CH₂-C), 2.47 (m, 2H, Ar-O-**CH**₂,), 1.85 (m, 2H, Ar-CH₂-**CH₂**-C(CH₂O-N-), 1.58 (m, 2H, Ar-O-CH₂-**CH₂**-(CH₂)₄-CH₃), 1.30 (m, 8H, Ar-O-CH₂-**CH₂**-(CH₂)₄), 0.82 (t, *J*=6.25Hz, 3H, Ar-O-(CH₂)₆-**CH₃**);

| | |
|---|---|
| **ESI (+):** | 333.9 ([M+H]⁺); |
| **TLC:** | R_{f} = 0. 48 (hexan/EtOAc:3/1) |
| **EI. Analysis:** | Calculated: C (72.04%), H (9.37%), N (4.20%); |
| | Found: C (71.94%), H (9.38%), N (4.15%); |

### Example 4

### Preparation of 7a-(4-(heptyloxy)phenethyl)tetrahydro-1H-oxazolo[3,4-c]oxazole

The compound ST-1071 was synthesized in the same manner as the compound ST-968. (see Example 2). ST-1071 was also obtained as HCl salt (ST-1071*HCl). Analytical data correspond to that of Example **3.**

**¹H NMR:** δ [ppm] (250 MHz, MeOD-*d₆*)

7.11 (d, *J*=8.5Hz, 2H, H_{Ar}), 6.87 (d, *J*=8.75Hz, 2H, H_{Ar}), 4.42 (d, *J*=5.75 Hz, 2H, -N-**CH₂**O-), 4.24 (d, *J*=5.75 Hz, 2H, -N-**CH₂**O-), 3.85 (t, *J*= 6.5Hz, Ar-O-**CH₂**-), 3.73 (d, *J*=8.75 Hz, 2H, CH₂-O-**CH₂**-C), 3.53 (d, *J*=8.75 Hz, 2H, CH₂-O-**CH₂**-C), 2.47 (m, 2H, Ar-O-**CH₂**,), 1.85 (m, 2H, Ar-CH₂-**CH₂**-C(CH₂O-N-), 1.58 (m, 2H, Ar-O-CH₂-**CH₂**-(CH₂)₄-CH₃), 1.30 (m, 8H, Ar-O-CH₂-CH₂-**(CH₂)₄**), 0.81 (t, *J*=6.25Hz, 3H, Ar-O-(CH₂)₆-**CH₃**);

| | |
|---|---|
| **ESI (+):** | 333.9 ([M+H]⁺); |
| **El. Analysis:** | Calculated: C (64.94%), H (8.72%), N (3.79%); |
| | Found: C (64.82%), H ( 8.95%), N (3.70%); |

### Example 5

### Methods for cell culture experiments

### Chemicals and materials:

IL-1β was from Cell Concept GmbH, Umkirch, Deutschland; VPC 23019 and W146 were from Avanti Polar, Alabaster, Alabama, US; pertussis toxin was from Merck Biosciences, Schwalbach, Germany; Hyperfilm MP and horseradish-coupled secondary antibodies were from GE Healthcare Systems, Freiburg, Germany; the monoclonal antibody against rat IIA-sPLA₂ was generated and characterized as previously described (Aarsman et al., 1989); the inducible NO synthase (iNOS) antibody was kindly provided by Prof. J. Pfeilschifter, University Hospital, Frankfurt am Main, Germany; the phospho-p42/p44 antibody, was from Cell Signalling, Frankfurt am Main, Germany; the total p42 and p44-MAPK antibodies were generated and characterized as previously described (Huwiler and Pfeilschifter, Br. J. Pharmacol. 113, 1455-1463); the PGE₂ enzyme-linked immunosorbent assay (ELISA) was from Assay Designs, BIOTREND Chemikalien GmbH, Köln, Germany; all cell culture nutrients were from Invitrogen/Life Technologies, Karlsruhe, Germany.

### Cell culture:

Rat renal mesangial cells were isolated, characterized, and cultured as previously described (Pfeilschifter et al., 1984). For the experiments performed in this study, cells between passages 8-30 were used. The human endothelial cell line EA.hy 926 was cultured in RPMI medium containing 10% (v/v) fetal bovine serum, 10mM Hepes, pH 7.4, 100 units/ml penicillin, and 100 µg/ml streptomycin. HCT-116 colon carcinoma cells were cultured in McCoy medium including 10% (v/v) fetal bovine serum, 100 units/ml penicillin, and 100 µg/ml streptomycin. Prior to stimulation, all cells were rendered quiescent for 24 h in DMEM including 0.1 mg/ml of fatty acid-free bovine serum albumin (BSA).

### Cell stimulation and Western blot analysis:

Confluent mesangial cells were stimulated as indicated in the figure legends in Dulbecco's modified Eagle medium (DMEM) supplemented with 0.1mg/ml of fatty acid-free bovine serum albumin and 10 mM HEPES. After stimulation, the supernatant was taken for PGE₂ or NO quantification and for detection of secreted IIA-sPLA₂. The cell monolayers were homogenized in lysis buffer and processed exactly as previously described (Huwiler et al., 2006). Cell lysates were taken for protein determination. Lysates containing equal amounts of protein, were separated by sodium dodecylsulfate-polyacrylamide gel electrophoresis (SDS-PAGE), transferred to nitrocellulose membranes and subjected for Western blot analysis using antibodies as indicated in the figure legends. Bands were detected by enhanced chemiluminescence method as recommended by the manufacturer.

### Detection of secreted IIA-sPLA₂:

Equal volumes of supernatants derived from the same number of cells were taken for protein precipitation using 7 % (w/v) of trichloroacetic acid. Precipitated proteins were redissolved in SDS-Laemmli buffer without dithiothreitol and subjected to SDS-PAGE (15 % acrylamide gel), transferred to nitrocellulose membranes and immunostained by using a monoclonal antibody against rat IIA-sPLA₂ at a dilution of 1:60 in 0.01 % milk powder.

### Determination of PGE₂ synthesis:

Confluent cells in 24-well plates were stimulated as indicated in the figure legends. 70µl of the supernatants were taken for a PGE₂ ELISA according to the manufacturer's instructions.

### Quantification of NO formation:

Quiescent mesangial cells in 24-well plates were stimulated as indicated, and the supernatants were taken for nitrite determination using Griess reagent as described (Green et al., 1982).

### Endothelial cell migration:

EA.hy 926 cells were cultured for 24 h under serum-free conditions. Chemotaxis was measured in Cell Culture Inserts (polycarbonate-coated membranes, 8 µM pore size). The lower compartment was filled with serum-free DMEM and 1.7 x 10⁵ cells were seeded per insert in the absence or presence of the indicated stimuli and inhibitors. After the indicated time periods, non-migrated cells were mechanically removed from the membrane and cells that had migrated into the membrane were fixed in 4% paraformaldehyde in PBS and stained with lug/ml of 4',6-diamidino-2-phenylindole (DAPI). DAPI-stained cells were counted using a fluorescence microscope. Each data point is the average number of cells in five random fields and is the mean of four individual wells.

### colony formation assay:

Cells were subcultured in 60 mm-diameter dishes at a density of 700 cells per dish. After 24 h, cells were treated with the indicated concentrations of the different compounds in growth medium and incubated for further 10 days to allow colony formation. Cells were then washed with PBS, air dried, and stained for 30 min with 2 % (w/v) crystal violet, washed with water and air dried again. The number of colonies was quantified using a ColCount^{™} (Mammalian Cell Colony Counter, Oxford Optronix, Oxford, U.K.). Only colonies containing more than 50 cells were evaluated.

*Statistical analysis:* Statistical analysis was performed by one way analysis of variance (ANOVA). For multiple comparisons with the same control group, the limit of significance was divided by the number of comparisons according to Bonferroni.

### Results

Since FTY720 is able to activate various signaling cascades in renal mesangial cells including the classical MAPK/ERK cascade (Xin et al., 2006), the inventors first determined whether the novel oxazolo-oxazole ST-968 had a similar effect. When using rat renal mesangial cells, it was indeed found that short-term stimulation for 10 min with ST-968 increased p42/44 phosphorylation which is considered to represent p42/44-MAPK activity (Fig. 1A and 1B). This effect was only partially blocked by pertussis toxin suggesting that a Gi/o protein is partially involved (Fig. 1A and B). In addition, the inventors used a recently developed S1P₁/S1P₃ receptor antagonist, i.e. VPC23019. This antagonist completely abolished the ST-968-induced p42/44 activation suggesting that the stimulatory effect of ST-968 is mediated by either the S1P₁ or S1P₃ receptor (or both) (Fig. 1A and B). In contrast, another SIP receptor antagonist, (R)-3-amino-(3-hexylphenylamino)-4-oxobutylphosphonic acid (W146), which was reported as a more S1P₁-selective antagonist (Sanna et al., 2006; Gonzales-Cabrera et al., 2008), had no effect on ST-968-triggered MAPK activation. ST-1071 also stimulated p42/44 phosphorylation after 10min in a concentration-dependent manner (Fig. 1C). However, it was found to be less potent than S1P (Fig. 1C).

These data prove for the first time that ST-968 and ST-1071 have agonistic potential on one or several of the SIP receptors and are able to activate signal transduction. As was shown that FTY720 is acting anti-inflammatory in mesangial cells by reducing the expression of cytokine-induced pro-inflammatory secretory phospholipase A₂ and subsequent PGE₂ synthesis (Xin et al., 2007), the inventors further investigated whether ST-968 exerted a similar effect.

Mesangial cells that were stimulated for 24h with interleukin-1β (IL-1β) plus forskolin showed a great induction of sPLA₂ protein expression and secretion from the cells into the supernatant (Fig. 2). In the presence of increasing doses of ST-968, the sPLA₂ expression is reduced thus, resembling the effect of FTY720.

Besides the PGE₂, also the nitric oxide (NO) is considered a pro-inflammatory mediator that is generated by the inducible NO synthase (iNOS) in mesangial cells under inflammatory conditions. The inventors found that the IL-1β/forskolin induced NO formation is dose-dependently reduced by ST-968. At 1-10µM of ST-968, a similar reduction was seen as obtained with 1-100nM of the glucocorticoid betamethasone (Fig. 3).

These data show that ST-968 is able to reduce pro-inflammatory mediator production and it is therefore likely that ST-968 also could exert anti-inflammatory and analgetic effects *in vivo.*

In a next step, the inventors investigated another cell system, i.e. the human endothelial cell line EA.hy 926. Also in this system, the inventors found that ST-968 could activate the p42/44-MAPK cascade. When compared to S1P, it was found that after 10min of stimulation, SIP was more potent to increase p42/44 phosphorylation than ST-968 (Fig. 4A and 4B). However, when extending the stimulation time period to 4h, ST-968 was more potently increasing p42/44 phosphorylation than SIP (Fig. 4A and 4B). These data indicate that SIP and ST-968 may have differential pharmacokinetic properties.

As it has been reported that in endothelial cells, SIP is regulating proliferation and migration of these cells and thereby may trigger angiogenesis under pathophysiological situations (Lucke and Levkau, Cell Physiol Biochem. 26, 87-96 (2010), the inventors further analyzed whether ST-968 and ST-1071 had an influence on the migration of human endothelial cells (cell line EA. hy 926) in an adapted Boyden chamber assay. As shown in Fig. 5, both ST-968 and ST-1071 were able to reduce serum-stimulated migration at 3µM and 10µM of concentration.

These results indicate that both compounds could be effective in treating pathophysiological conditions associated with increased endothelial cell migration such as cancer and various forms of retinopathies including diabetic retinopathy and age-related macula degeneration.

In a third approach, the inventors used the human colon cancer cell line HCT-116. Previously, it had been shown that FTY720 can exert anti-tumorigenic effects (Wang et al., 1999; Azuma et al., 2002; LaMontagne et al., 2006). The inventors therefore tested here whether ST-968 and ST-1071, (and a series of other FTY720 derivatives), affected the colony forming capacity of cancer cells. As seen in Fig. 6, FTY720 completely abolished colony formation in these cancer cells whereas the FTY720 precursor myriocin, had no effect. Also the compounds ST-968 and ST-1071 were active and strongly reduced colony formation (Fig. 6).

### References:

Azuma H, Takahara S, Ichimaru N, Wang JD, Itoh Y, Otsuki Y, Morimoto J, Fukui R, Hoshiga M, Ishihara T, Nonomura N, Suzuki S, Okuyama A, Katsuoka Y. (2002) Marked prevention of tumor growth and metastasis by a novel immunosuppressive agent, FTY720, in mouse breast cancer models. Cancer Res. 2002, 1;62(5):1410-9.
Berge, S. M., Bighley, L. D., Monkhouse, D. C. Pharmaceutical salts, J. Pharm. Sci. 1977, 66(1), 1-19.
Darabantu, M., Ple, G., Maiereanu, C., Silaghi-Dumitrescu, I., Ramondenc, Y., Mager, S. Synthesis and Stereochemistry of Some 1,3-Oxazolidine Systems Based on TRIS (α,α,α-Trimethylolaminomethane) and Related Aminopolyols Skeleton. Part 1: (Di)spiro-1,3-oxazolidines , Tetrahedron, 2000b, 56(23), 3785-3798.
Darabantu, M., Ple, G., Maiereanu, C., Silaghi-Dumitrescu, I., Ramondenc, Y., Mager, S. Synthesis and Stereochemistry of Some 1,3-Oxazolidine Systems Based on TRIS (α,α,α-trimethylolaminomethane) and Related Aminopolyols Skeleton. Part 2: 1-Aza-3,7-dioxabicyclo[3.3.0]octanes , Tetrahedron, 2000b, 56(23), 3799-3816.
Darabantu, M., Maiereanu, C., Silaghi-Dumitrescu, I., Toupet, L., Condamine, E., Ramondenc, Y., Berghian, C., Ple, G., Ple, N. 3,7-Dioxa-1-azabicyclo[3.3.0]octanes Substituted at the C-5 Position - From Local to Global Stereochemistry, Eur. J. Org. Chem., 2004, 12, 2644-2661.
Eyster, K. M., Die Membrane und die Lipide als integrale Teilnehmer an Signaltransduction, 2007, Adv. Physiol. Edu. 31, 5-16.
Fernandis, A., Wenk, M. R.,Membrane lipids as signaling molecules, Curr. Opin. Lipidol., 2007 , 18(2), 121-8.
Gonzalez-Cabrera PJ, Jo E, Sanna MG, Brown S, Leaf N, Marsolais D, Schaeffer MT, Chapman J, Cameron M, Guerrero M, Roberts E, Rosen H. (2008) Full pharmacological efficacy of a novel S1P1 agonist that does not require S1P-like headgroup interactions. Mol. Pharmacol. 2008;74(5):1308-18.
Gómez-Muñoz, A., Modulation of cell signalling by ceramides, Biochim. Biophys. Acta, 1998, 1391, 92-109.
Kiuchi, M., Adachi, K., Kohara, T., Minoguchi, M., Hanano, T., Aoki, Y., Mishina, T., Arita, M., Nakao, N., Ohtsuki, M., Hoshino, Y., Teshima, K., Chiba, K., Sasaki, S., Fujita, T. Synthesis and Immunosuppressive Activity of 2-Substituted 2-Aminopropane-1,3-diols and 2-Aminoethanols, J. Med. Chem. 2000, 43, 2946-2961.
LaMontagne K, Littlewood-Evans A, Schnell C, O'Reilly T, Wyder L, Sanchez T, Probst B, Butler J, Wood A, Liau G, Billy E, Theuer A, Hla T, Wood J. (2006) Antagonism of sphingosine-1-phosphate receptors by FTY720 inhibits angiogenesis and tumor vascularization. Cancer Res. 2006, 1;66(1):221-31.
Levade, T., Jaffrezou, J.-P. Signalling sphingomyelinases: which, where, how and why?, Biochim Biophys Acta, 1999, 19,1438, 1-17.
Mathias, S., Pena, L. A., Kolesnick, R. N. Signal transduction of stress via ceramide, Biochem. J., 1998, 335, 465-80.
Riboni, L., Viani, P., Bassi, R., Prinetti, a., Tettamanti, G. The role of sphingolipids in the process of signal transduction , Prog. Lipid Res. 1997, 36, 153-195.
Sanna MG, Wang SK, Gonzalez-Cabrera PJ, Don A, Marsolais D, Matheu MP, Wei SH, Parker I, Jo E, Cheng WC, Cahalan MD, Wong CH, Rosen H. (2006) Enhancement of capillary leakage and restoration of lymphocyte egress by a chiral S1P1 antagonist in vivo. Nat. Chem. Biol. 2006;2(8):434-41.
Stahl, P. H., Wermuth, C. G. Handbook of pharmaceutical salts: Properties, selection and use. 2002, Verlag Helvetica Chimica Acta, Zürich; ISBN: 3-906390-26-8.
Wang JD, Takahara S, Nonomura N, Ichimaru N, Toki K, Azuma H, Matsumiya K, Okuyama A, Suzuki S. (1999) Early induction of apoptosis in androgen-independent prostate cancer cell line by FTY720 requires caspase-3 activation. Prostate. 1999, 15;40(1):50-5.
Wang L, Lee JF, Lin CY, Lee MJ (2008) Rho GTPases mediated integrin alpha v beta 3 activation in sphingosine-1-phosphate stimulated chemotaxis of endothelial cells. Histochem. Cell Biol. 2008;129(5):579-88.
Xin C, Ren S, Eberhardt W, Pfeilschifter J, Huwiler A. (2006) The immunomodulator FTY720 and its phosphorylated derivative activate the Smad signalling cascade and upregulate connective tissue growth factor and collagen type IV expression in renal mesangial cells. Br. J. Pharmacol. 2006;147(2):164-74.
Xin C, Ren S, Eberhardt W, Pfeilschifter J, Huwiler A. FTY720 suppresses interleukin-1beta-induced secretory phospholipase A2 expression in renal mesangial cells by a transcriptional mechanism. Br. J. Pharmacol. 2007 Apr; 150(7):943-50.
Zivkovic, A., Stark, H., Efficient chromatography-free synthesis of the oxy-analogue of Fingolimod, Tetrahedron Letters, 2010, 51(29), 3769-3771.

## Claims

1. A compound of formula (I): 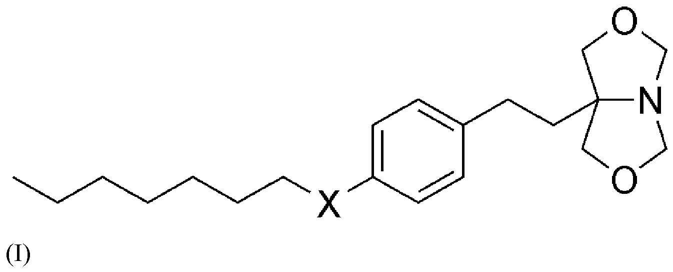 wherein X is selected from a methylene group (CH₂) and oxygen (O);
and pharmaceutically acceptable salts thereof.

2. The compound according to claim 1, wherein X is CH₂.

3. The compound according to claim 1 wherein X is O.

4. A pharmaceutical composition comprising as active ingredient a compound according to any of claims 1 to 3, and optionally further comprising pharmaceutically acceptable carrier, adjuvant or diluent.

5. The compound according to any of claims 1 to 3 or the pharmaceutical composition according to claim 4 for the treatment of diseases.

6. The pharmaceutical composition according to claim 4 for use in the treatment of a disorder selected from the group consisting of proliferative disorders, neurodegenerative disorders, metabolism-associated conditions, infectious diseases, immunity-associated conditions, and other pathological states that are **characterized by** increased prostaglandins formation.

7. The pharmaceutical composition according to claim 6, wherein said proliferative disorder is cancer, or wherein said angiogenesis-associated disorder is selected from diabetic retinophaty and macula degeneration, or wherein said neurodegenerative disorder is Alzheimer's disease or multiple sclerosis, or wherein said metabolism-associated condition is selected from diabetes, cystic fibrosis, and lipid storage diseases, or wherein said infectious disease is selected from the group consisting of viral infections, bacterial infections, fungal infections, and protozoal infections, or wherein said immunity-associated disorders are autoimmune diseases or inflammatory diseases graft versus host disease (GVHD) or allergy, or wherein said pathological states associated with increased prostaglandin formation is pain.

8. The pharmaceutical composition according to claim 6 or 7 for use in killing of drug-sensitive and drug-resistant cancer cells.

9. Use of a compound according to any one of claims 1 to 3 in the preparation of a medicament for treating a disorder selected from the group consisting of proliferative disorders, angiogenesis-associated disorder, neurodegenerative disorders, metabolism-associated conditions, infectious diseases, immunity-associated conditions, and pain.
